# EUROPEAN PATENT APPLICATION

(11) **EP 2 123 269 A1**
(43) Date of publication of application: **25.11.2009**
(21) Application number: 08009414.7
(22) Date of filing: 21.05.2008
(51) Int. Cl.: A61K 31/185, A61K 33/24, A61K 47/48, A61P 37/00, A61P 17/06

(54) **Nanoparticles functionalized with sulfated amino alcohols for the inhibition of selectin-mediated cell adhesion**

(71) Applicant: Freie Universität Berlin, 14195 Berlin (DE); Charité - Universitätsmedizin Berlin, 10117 Berlin (DE)
(72) Inventor: Schlecht, Sabine, 10963 Berlin (DE); Dernedde, Jens, 10629 Berlin (DE); Reissig, Hans-Ulrich, 12165 Berlin (DE); Roskamp, Meike, 10717 Berlin (DE); Enders, Sven, 10598 Berlin (DE)
(74) Representative: Engelhard, Markus

(57) **Abstract**

The present invention relates to functionalized nanoparticles inhibiting selectin-mediated cell adhesion and their use as anti-inflammatory therapeutics. More specifically, said functionalized nanoparticles comprise a core, formed by gold, a shell coating said core, formed by a monolayer of a linker molecule, and an acyclic or carbocyclic sulfated amino alcohol linked to said linker molecule via a peptide bond.

## Description

The present invention relates to functionalized nanoparticles inhibiting selectin-mediated cell adhesion and their use as anti-inflammatory therapeutics. More specifically, said functionalized nanoparticles comprise a core, formed by gold, a shell coating said core, formed by a monolayer of a linker molecule, and an acyclic or carbocyclic sulfated amino alcohol linked to said linker molecule via a peptide bond.

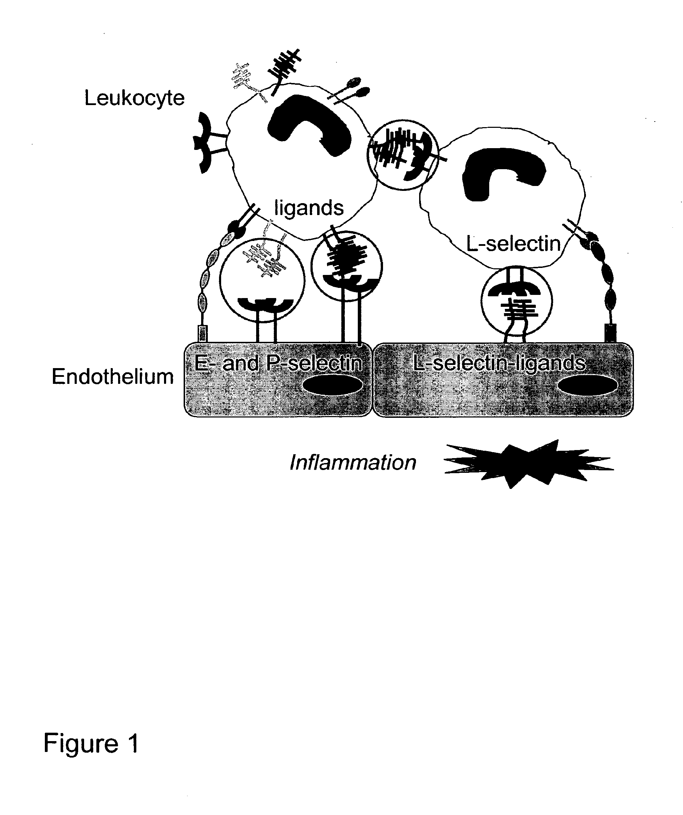

Recruitment of white blood cells (leukocytes) to the endothelial cells lining blood vessels and their subsequent emigration into the adjoining tissue are observed in all forms of the inflammatory response (see **Figure 1**). Initial contact and rolling along the endothelial surface is mediated by transient receptor-ligand interactions between the three selectins and their ligands (Ley K., Trends Mol. Med. 9 (2003) 2638). Tight contact between the leukocytes and the endothelium is subsequently achieved by the interaction of activated integrins with adhesion molecules of the immunoglobulin superfamily (Springer T.A., Nature 346 (1990) 425-434). In addition to desirable defensive effects and repair of tissue defects the uncontrolled emigration of leukocytes from the bloodstream can also be of pathological importance and may lead to tissue damage (Lefer D.J., Annu. Rev. Pharmacol. Toxicol. 40 (2000) 283-294). The general involvement of endothelial cell adhesion molecules in acute and chronic inflammatory processes thus makes them to suitable targets for diagnosis and therapy (Boehncke W.H. et al., Exp. Dermatol. 14 (2005) 70-80).

E- and P-selectin as well as ligands of L-selectin are inflammatory-dependently expressed on the microvascular endothelium, whereas L-selectin is presented by leukocytes (Ley K., Trends Mol. Med. 9 (2003) 2638; Springer T.A., Nature 346 (1990) 425-434). Findings that the tetrasaccharide Sialyl Lewis X (sLex) is a crucial binding partner of selectins and that polyvalency is a key for the targeted blockade of leukocyte adhesion are well-established and have formed the basis for the development of various selectin inhibitors (Simanek et al., Chem. Rev. 98 (1998) 833-862). However, targeting selectin has not yet lead to the development of market-ready therapeutics despite the fact that high-affinity inhibitors are at hand (Simanek et al., Chem. Rev. 98 (1998) 833-862).

Currently, therapeutic intervention in the case of rheumatoid arthritis is achieved by the use of inhibitors of the inflammatory cytokine TNFα (Infliximab, Etanercept). The anti-integrin antibody Efalizumab is on the market and approved for the systemic therapy in the case of psoriasis. Other substances are currently tested in clinical trials, such as the substance Bimosiamose (Revotar, Henningsdorf), a pan-selectin antagonist, which belongs to the class of small molecule drugs and is supposed to be used for asthma, psoriasis, reperfusion damages.

Linear neoglycopolymers carrying sulfated sLex structures as well as sulfated dendritic polyethylene oxide (PEO) glycopolymers have been described before and can achieve IC₅₀ values in the lower nanomolar range (Simanek et al., Chem. Rev. 98 (1998) 833-862; Mowery et al., Chem. Biol. 11 (2004) 725-732; Rele et al., J. Am. Chem. Soc. 127 (2005) 10132-10133). Functionalized nanoparticles, in particular glyco-nanoparticles with a core of polymer or gold, have so far been mainly used with respect to diagnostic and imaging applications. Their potential therapeutic application is currently being tested using animal models (John et al., FASEB J. 17 (2003) 2296-2298; Rojo et al., ChemBioChem 5 (2004) 291-297). Various carbohydrate derivatives or carbohydrate mimetics analogous to sLex structures have been tested as ligands of selectin as well (Wong et al., Chem. Rev. 98 (1998) 833-863; Kaila et al., J. Med. Chem. 48 (2005) 4346-4357).

It was an object of the present invention to provide for a high-affinity inhibitor of selectin-ligand interactions, which is suitable for the treatment of diseases, particularly inflammatory diseases. It was another object of the present invention to provide for a high-affinity inhibitor that can be easily synthesized and is non-toxic as well as stable in a physiological environment.

The objects of the present invention are solved by a functionalized nanoparticle, comprising a core, formed by gold,
a shell coating said core, formed by a monolayer of a linker molecule, and
an acyclic or carbocyclic sulfated amino alcohol linked to said linker molecule via a peptide bond.

In one embodiment said core has a diameter ≤ 50 nm, preferably ≤ 25 nm, more preferably ≤ 15 nm.

In one embodiment said linker molecule is a linear or branched, preferably a linear alkyl chain, with at least one binding functionality for the attachment of said linker molecule to said core.

In one embodiment said alkyl chain has 5 to 26 C-atoms, preferably 10 to 26 C-atoms.

In one embodiment said binding functionality comprises at least one sulfur atom.

In a preferred embodiment said binding functionality is selected from the group comprising thiol (SH), thiolate (S⁻) and disulfide (S₂).

In one embodiment of the present invention said acyclic or carbocyclic sulfated amino alcohol has the general formula 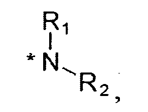 wherein
* denotes the nitrogen atom that is linked to a carbonyl group forming said peptide bond; and R₁ and R₂ are independently selected from the group comprising H and molecules having the general formula 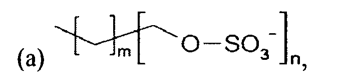 with m and n being integer numbers between and including 1 and 3, 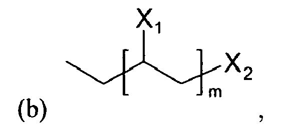 with m being an integer number between and including 1 and 5, and
   X₁ and X₂ being independently selected from the group comprising H, OSO₃⁻, and CH₂OSO₃⁻, 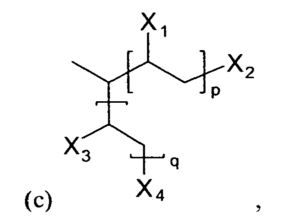 with p and q being integer numbers between and including 1 and 5, and X₁ X₂, X₃, and X₄ being independently selected from the group comprising H, OSO₃⁻, and CH₂OSO₃⁻, and 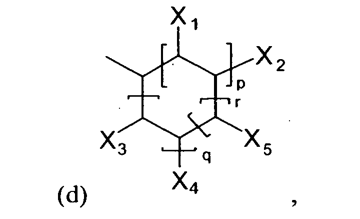 with p and q being integer numbers between and including 1 and 5, r being 0 or 1, and X₁ X₂, X₃, X₄, and X₅ being independently selected from the group comprising H, OSO₃⁻, and CH₂OSO₃⁻,
wherein at least one of R₁ and R₂ comprises at least one sulfated alcohol group selected from OSO₃⁻ and CH₂OSO₃⁻,

In one embodiment said acyclic or carbocyclic sulfated amino alcohol is acyclic and preferably selected from the group comprising sulfated ethanolamine, sulfated serinol, and sulfated tromethamine.

In one embodiment said linker molecule is represented by the formula 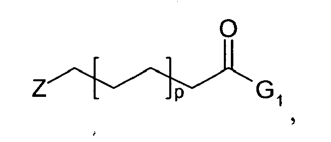 wherein
Z represents a binding functionality as defined above, preferably selected from the group comprising SH, S⁻, and S₂,
p is 4-12, and
G₁ is an acyclic or carbocyclic sulfated amino alcohol, preferably an acyclic or carbocyclic sulfated amino alcohol as defined above.

The objects of the present invention are also solved by a method for producing a functionalized nanoparticle as defined above, comprising the linking of a sulfated amino alcohol via a peptide bond to linker molecules bound to a gold core.

The objects of the present invention are also solved by a nanoparticle as defined above for the treatment of diseases.

The objects of the present invention are also solved by the use of a nanoparticle as defined above for the production of a medicament for the treatment of inflammatory diseases.

In one embodiment said inflammatory diseases are chronic inflammatory diseases, in particular rheumatoid arthritis, asthma, and psoriasis.

In one embodiment said inflammatory diseases are ischemia reperfusion damages or graft repulsion.

The objects of the present invention are also solved by the use of a nanoparticle as defined above as selectin inhibitor.

In a preferred embodiment the nanoparticle as defined above is used as inhibitor of L-selectin and/or P-selectin and/or E-selectin.

The term "functionalized nanoparticle" as used herein is meant to refer to a nanoparticle that is provided with (or coordinated with) functional groups or molecules that allow the binding to a specific target, such as a protein, preferably a specifically selected protein, such as a selectin, e.g. an L-, P-, or E-selectin, or a nanoparticle that is provided with functional groups or molecules that increase the stability of the nanoparticle in a given environment, e.g. by preventing aggregation.

The term "core" as used herein is meant to refer to a core or nucleus that provides a base for the attachment of linker molecules via functional groups, such as thiol groups.

The term "shell" as used herein is meant to refer to a shell covering the above-defined core and being formed by a monolayer, preferably a closed monolayer of a plurality of linker molecules. For example, in one embodiment of the present invention a core with a diameter of 6 nm is coated by a monolayer formed by approximately 1300 linker molecules.

The term "linker molecule" as used herein is meant to refer to a molecule, which is able to bind to the above-defined core via at least one terminal or non-terminal binding functionality, such as a thiol group, and which is provided with at least one additional polar functional group, that allows for binding to a target protein, such as selectin.

The term "selectin inhibitor" as used herein is meant to refer to exogenous molecules or compounds, such as the nanoparticles described herein, that are able to bind to selectins with high affinity, thereby disrupting or preventing physiological selectin-ligand interactions.

The inventors have surprisingly found that functionalized nanoparticles according to the present invention are excellent inhibitors of selectin-ligand interactions. With half inhibitory concentrations (IC₅₀) in the low picomolar range, they have a far higher affinity for selectins than any other known selectin inhibitor. By efficiently inhibiting selectin-ligand interactions, which in turn mediate the interaction between leukocytes and endothelial cells, the functionalized nanoparticles according to the present invention could provide a potent way of treating a variety of inflammatory diseases.
Nanoparticles according to the present invention, i.e. comprising an acyclic or carbocyclic sulfated amino alcohol, show a high stability with respect to aggregation at physiological pH values, even at concentrations in the high nanomolar range. The covalent linking of the acyclic or carbocyclic sulfated amino alcohols to the linker molecules of the shell via peptide bonds provides for hydrolytic stability. Furthermore, the inventors show that the functionalized nanoparticles according to the present invention are non-toxic for living cells.

Reference is now made to the figures and tables, wherein
**Figure 1** is a schematic illustration of the interactions between endothelial and leukocyte adhesion molecules during leukocyte adhesion. Circles highlight selectin-ligand interactions;

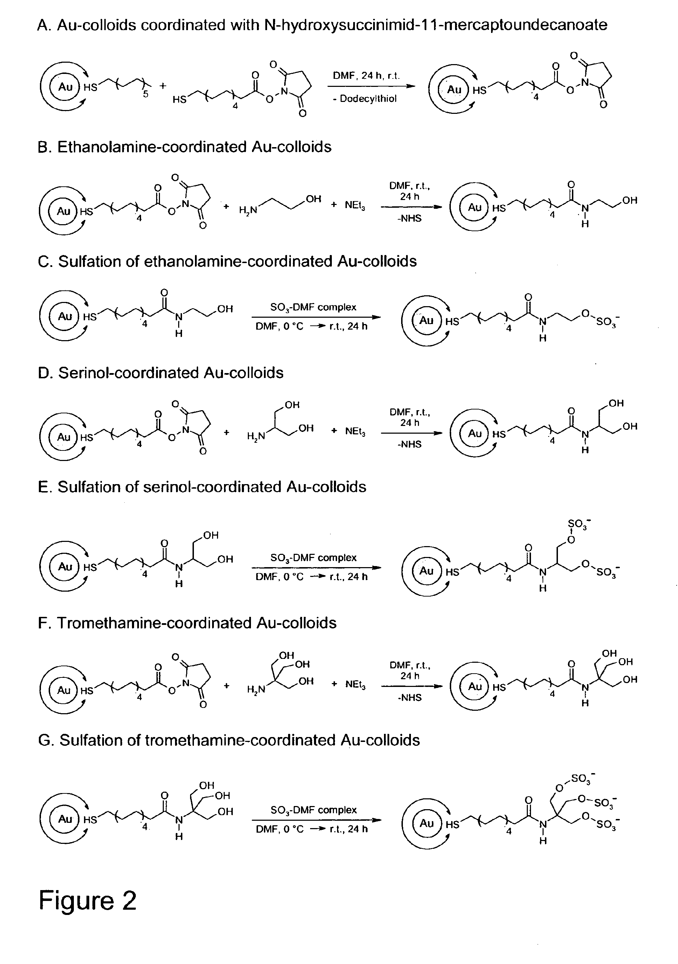

**Figure 2** shows the syntheses of various functionalized gold nanoparticles. **Figure 2A** shows the synthesis of gold colloids covered/coordinated with N-hydrosuccinimid-11-mercaptoundecanoate, **Figure 2B** shows the synthesis of ethanolamine-coordinated Au-colloids, **Figure 2C** shows the sulfation of ethanolamine-coordinated Au-colloids, **Figure 2D** shows the synthesis of serinol-coordinated Au-colloids, **Figure 2E** shows the sulfation of serinol-coordinated Au-colloids, **Figure 2F** shows the synthesis of tromethamine-coordinated Au-colloids, and **Figure 2G** shows the sulfation of tromethamine-coordinated Au-colloids;
**Figure 3** is a graph showing the *in vitro* inhibition of L-selectin/P-selectin-ligand interaction by functionalized gold (Au)-colloids according to the present invention, as determined by surface plasmon resonance (SPR);
**Table 1** shows the half inhibitory concentrations (IC₅₀) of three different functionalized nanoparticles according to the present invention for both L-selectin and P-selectin; and
**Table 2** shows the vitality of cells treated with various concentrations of serinol-disulfate-functionalized Au-nanoparticles.

The invention is now further described by reference to the following examples which are intended to illustrate, not to limit the scope of the invention.

### Example 1: Au-colloids coordinated with N-hydroxysuccinimid-11-mercaptoundecanoate

Under stirring, a solution of dodecylthiol-coordinated Au-colloids (6 nmol, d = 6.2 nm) in 4 ml of chloroform was dropped into a solution of 11-mercaptoundecanoic acid-N-hydroxysuccinimidester (0.189 g, 0.6 mmol, 10⁵ eq) in 40 ml of anhydrous DMF. After 10 minutes, the solution was concentrated to 30 ml and stirred at room temperature (r.t.) for another 24 h. Then, the particle solution was dialysed against DMF (MWCO: 4000-6000, three times for 3 h with 200 ml of DMF). After transfer into anhydrous DMF the resulting red solution was stable for several days at 4°C **(****Figure 2A****).**
¹H-NMR (400 MHz, DMSO-d₆): 2.79 (*s,* 4H), 2.64 (*m,* 2H), 2.2 (*t,* 2H), 1.8-1.4 (*m,* 4H), 1.4-1.15 (*m*, 12H).

### Example 2: Ethanolamine-coordinated Au-colloids

Under stirring, ethanolamine (9 µl, 0.15 mmol) was added to a solution of 11-mercaptoundecanoic acid-N-hydroxysuccinimidester-coordinated Au-colloids (1 nmol NP, d = 6.2 nm; see Example 1) in 5 ml of anhydrous DMF. The reaction mixture was stirred for 30 minutes before adding triethylamine (20 µl, 0.14 mmol). After further 24 h of stirring at r.t. the nanoparticle solution was dialysed against DMF (MWCO: 4000-6000, three times for 3 h with 200 ml of DMF), dried under vacuum and resolved in 5 ml of anhydrous DMF (**Figure 2B**).

### Example 3: Sulfation of ethanolamine-coordinated Au-colloids

At 0°C and under stirring, a solution of SO₃·DMF (300 mg, 2.0 mmol) in 1 ml of anhydrous DMF was dropped slowly into a solution of ethanolamine-coordinated Au-colloids (1 nmol NP, d = 6.2 nm; see Example 2) in 10 ml of anhydrous DMF. After 24 h of stirring at r.t., the nanoparticle solution was dialysed first against DMF (MWCO: 4000-6000, three times for 3 h with 200 ml of DMF), then against millipore water (MWCO: 4000-6000, three times for 3 h with 200 ml of H₂O). The resulting red solution was stable at 4°C for several months **(****Figure 2C****).**
¹H-NMR (400 MHz, D₂O c_{NP} = 1 µM): 1.25 (bs), 1.56 (bs), 2.22 (bs, *CH₂-*CONH), 2.69 (bs, *CH₂-*SH), 3.45 (bs, *CH₂-*NHCO), 4.06 (bs, *CH₂-*OSO₃⁻)
ATR-IR: 3302 cm⁻¹ (*s,* ν(N-H)), 2918 cm⁻¹ and 2850 cm⁻¹ (*s, ν*(C-H)), 1686 and 1639 cm⁻¹ (*s,* ν(C=O)), 1532 cm⁻¹ (s, δ(N-H)), 1465 - 1452 cm⁻¹ and 1211 cm⁻¹ (*s*, R-O-SO₂-OR'), 1066 cm⁻¹ (*s*), 1020 cm⁻¹ (*s*), 959 cm⁻¹ (w), 719 cm⁻¹ (s), 579 cm⁻¹ (s).

### Example 4: Serinol-coordinated Au-colloids

Under stirring, a solution of serinol (9 mg, 0.1 mmol) in 1 ml of anhydrous DMF was added to a solution of 11-mercaptoundecanoic acid-N-hydroxysuccinimidester-coordinated Au-colloids (1 nmol NP, d = 6.2 nm; see Example 1) in 5 ml of anhydrous DMF. The reaction mixture was stirred for 30 minutes before adding triethylamine (20 µl, 0.14 mmol). After further 24 h of stirring at r.t., the nanoparticle solution was dialysed against DMF (MWCO: 4000-6000, three times for 3 h with 200 ml of DMF), dried under vacuum and resolved in 5 ml of anhydrous DMF (**Figure 2D**).

### Example 5: Sulfation of serinol-coordinated Au-colloids

At 0°C and under stirring, a solution of SO₃·DMF (154 mg, 1.0 mmol) in 1 ml of anhydrous DMF was dropped slowly into a solution of serinol-coordinated Au-colloids (1 nmol NP, d = 6.2 nm; see Example 4) in 10 ml of anhydrous DMF. After 24 h of stirring at r.t., the nanoparticle solution was dialysed first against DMF (MWCO: 4000-6000, three times for 3 h with 200 ml of DMF), then against millipore water (MWCO: 4000-6000, three times for 3 h with 200 ml of H₂O). The resulting red solution was stable at 4°C for several months (**Figure 2E**).
¹H-NMR (400 MHz, D₂O): 1.32 (bs), 1.62 (bs), 2.30 (bs, *CH₂*-CONH), 2.89 (bs, *CH₂-*SH), 4.15 (bs, *CH₂*-OSO₃⁻), 4.43 (bs, *CH-*(CH₂OSO₃⁻)₃.
ATR-IR: 3482 und 3294 cm⁻¹ (s, v(O-H)), 3039 cm⁻¹ (w), 2924 cm⁻¹ and 2853 cm⁻¹ (*s,* ν(C-H)), 1683 and 1653 cm⁻¹ (*s*, *ν*(C=O)), 1539 cm⁻¹ (s, δ(N-H)), 1490 cm⁻¹ (s), 1465 - 1453 cm⁻¹ and 1218 cm⁻¹ (*s*, R-O-SO₂-OR'), 1004 cm⁻¹ (*s*), 950 cm⁻¹ (s), 788 cm⁻¹ (s), 577 cm⁻¹ (s).

### Example 6: Tromethamine-coordinated Au-colloids

Under stirring, a solution of tromethamine (18.2 mg, 0.15 mmol) in 1 ml of anhydrous DMF was added to a solution of 11-mercaptoundecanoic acid-N-hydroxysuccinimidester-coordinated Au-colloids (1 nmol NP, d = 6.2 nm; see Example 1) in 5 ml of anhydrous DMF. The reaction mixture was stirred for 30 minutes before adding triethylamine (80 µl, 0.56 mmol). After further 24 h of stirring at r.t., the nanoparticle solution was dialysed against DMF (MWCO: 4000-6000, three times for 3 h with 200 ml of DMF), dried under vacuum and resolved in 5 ml of anhydrous DMF (**Figure 2F**).

### Example 7: Sulfation of tromethamine-coordinated Au-colloids

At 0°C and under stirring, a solution of SO₃-DMF (300 mg, 2.0 mmol) in 1 ml of anhydrous DMF was dropped slowly into a solution of tromethamine-coordinated Au-colloids (1 nmol NP, d = 6.2 nm; see Example 6) in 10 ml of anhydrous DMF. After 24 h of stirring at r.t., the nanoparticle solution was dialysed first against DMF (MWCO: 4000-6000, three times for 3 h with 200 ml of DMF), then against millipore water (MWCO: 4000-6000, three times for 3 h with 200 ml of H₂O). The resulting red solution was stable at 4°C for several months (**Figure 2G**).
¹H-NMR (400 MHz, D₂O, c_{NP} = 1 µM): 1.29 (bs), 1.59 (bs), 2.24 (bs, *CH₂-*S), 2.90 (bs), 4.3 (bs, *CH₂-*O-SO₃⁻).
ATR-IR: 3291 cm⁻¹(*w*, *ν*(N-H)), 2920 cm⁻¹ and 2851 (*s*, *ν*(C-H)), 2465 (*w*, *ν*(S-H)), 1682 and 1633 cm⁻¹ (*s*, *ν*(C=O)), 1528 cm⁻¹ (s, δ(N-H)), 1466 - 1453 cm⁻¹ and 1210 cm⁻¹ (*s*, R-O-SO₂-OR'), 1002 cm⁻¹ (*s*), 801- 774 cm⁻¹ (s), 578 cm⁻¹ (s).

### Example 8: In vitro inhibition of selectin-ligand interaction using functionalized colloidal gold nanoparticles

Au-nanoparticles (d = 6.2 nm) functionalized with sulfated enthanolamine, sulfated serinol, and sulfated tromethamine were tested in a competitive *in vitro* L-selectin- and P-selectin-ligand binding assay in order to determine their half inhibitory concentrations (IC₅₀).

Selectin-ligand binding was analyzed by surface plasmon resonance (SPR). First, the binding (detected as resonance units) of selectin-coated nanoparticles to a selectin ligand, immobilized on a sensor chip, was tested, and the resulting signal was henceforth referred to as 100% binding. Preincubation of the selectin-coated particles with varying concentrations of a selectin inhibitor decreased the binding signal. The calculated IC₅₀ value (half inhibitory concentration) is the molar concentration of the inhibitor needed to reduce the binding signal to 50% of the initial value.

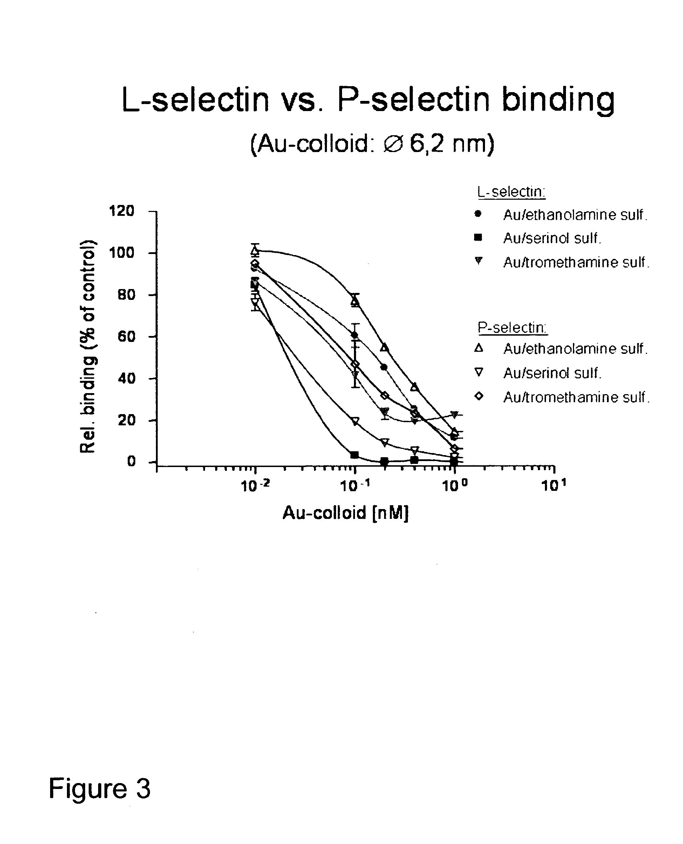

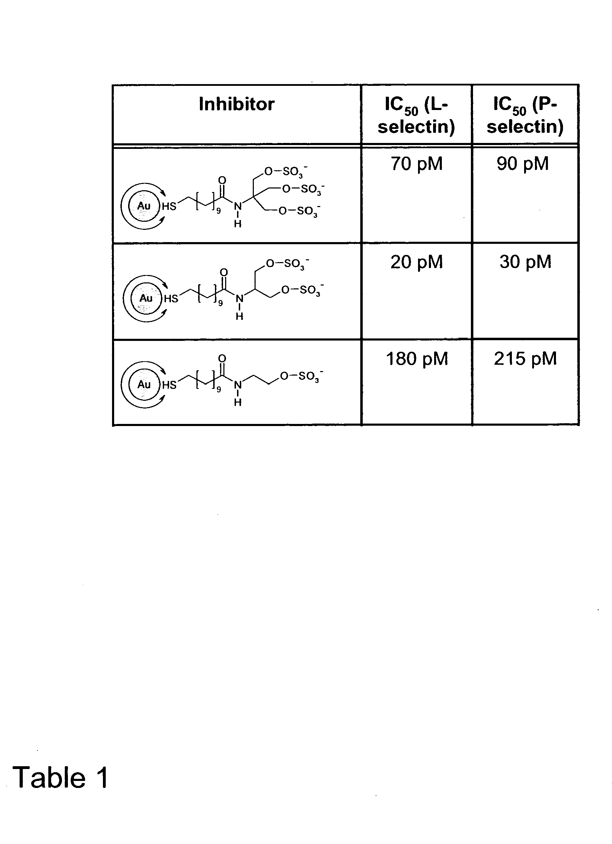

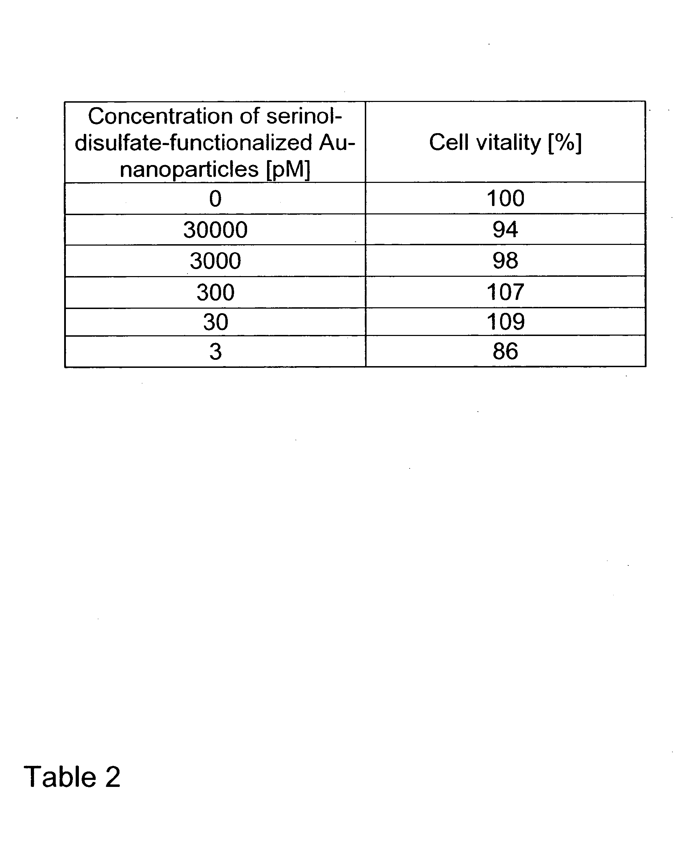

As shown in **Figure 3** and **Table 1**, all three nanoparticles inhibited L-selectin- and P-selectin-ligand interaction at concentrations of the picomolar range.

### Example 9: Cytotoxicity

Cytotoxicity of Serinol-disulfat-functionalized Au-nanoparticles (having concentrations between 3 and 30000 pM) was examined in cultures of the lymphocyte cell line Jurkat expressing L-selectin. The analysis was performed using a Cell Titer 96AQᵤₑₒᵤₛ One Solution Cell Proliferation Assay (Promega). As shown in **Table 2,** cell proliferation was only marginally influenced in comparison to the untreated control cells (vitality = 100%). Interestingly, there seemed to be a concentration range, where cell vitality was even positively influenced (vitality > 100%).

## Claims

1. Functionalized nanoparticle, comprising
a core, formed by gold,
a shell coating said core, formed by a monolayer of a linker molecule, and
an acyclic or carbocyclic sulfated amino alcohol linked to said linker molecule via a peptide bond.

2. Nanoparticle according to claim 1, wherein said core has a diameter ≤ 50 nm, preferably ≤ 25 nm, more preferably ≤ 15 nm.

3. Nanoparticle according to claim 1 or 2, wherein said linker molecule is a linear or branched, preferably a linear alkyl chain, with at least one binding functionality for the attachment of said linker molecule to said core.

4. Nanoparticle according to claim 3, wherein said alkyl chain has 5 to 26 C-atoms, preferably 10 to 26 C-atoms.

5. Nanoparticle according to claim 3 or 4, wherein said binding functionality comprises at least one sulfur atom.

6. Nanoparticle according to claim 5, wherein said binding functionality is selected from the group comprising thiol (SH), thiolate (S⁻) and disulfide (S₂).

7. Nanoparticle according to any of claims 1 to 6, wherein said acyclic or carbocyclic sulfated amino alcohol has the general formula 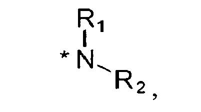 wherein
* denotes the nitrogen atom that is linked to a carbonyl group forming said peptide bond; and
R₁ and R₂ are independently selected from the group comprising H and molecules having the general formula 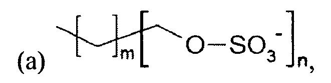 with m and n being integer numbers between and including 1 and 3, 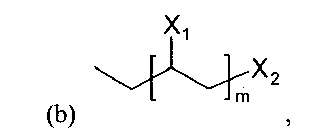 with m being an integer number between and including 1 and 5, and X₁ and X₂ being independently selected from the group comprising H, OSO₃⁻, and CH₂OSO₃⁻, 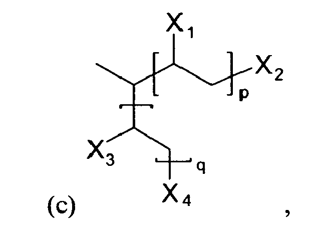 with p and q being integer numbers between and including 1 and 5, and X₁, X₂, X₃, and X₄ being independently selected from the group comprising H, OSO₃⁻, and CH₂OSO₃⁻, and 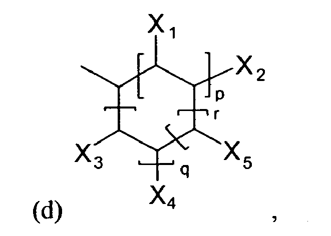 with p and q being integer numbers between and including 1 and 5, r being 0 or 1, and
X₁, X₂, X₃, X₄, and X₅ being independently selected from the group comprising H, OSO₃⁻, and CH₂OSO₃⁻,
wherein at least one of R₁ and R₂ comprises at least one sulfated alcohol group selected from OSO₃⁻ and CH₂OSO₃⁻.

8. Nanoparticle according to any of the foregoing claims, wherein said linker molecule is represented by the formula 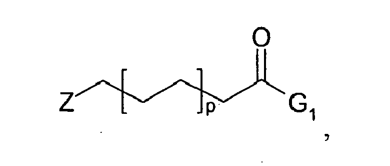 wherein
Z represents a binding functionality as defined in any of claims 3, 5, and 6, preferably selected from the group comprising SH, S⁻, and S₂,
p is 4-12, and
G₁ is an acyclic or carbocyclic sulfated amino alcohol, preferably an acyclic or carbocyclic sulfated amino alcohol as defined in claim 7.

9. Method for producing a functionalized nanoparticle according to any of claims 1 to 8, comprising the linking of a sulfated amino alcohol via a peptide bond to linker molecules bound to a gold core.

10. Nanoparticle according to any of claims 1 to 8 for the treatment of diseases.

11. Use of a nanoparticle according to any of claims 1 to 8 for the production of a medicament for the treatment of inflammatory diseases.

12. Use according to claim 11, wherein said inflammatory diseases are chronic inflammatory diseases, in particular rheumatoid arthritis, asthma, and psoriasis.

13. Use according to claim 11, wherein said inflammatory diseases are ischemia reperfusion damages or graft repulsion.

14. Use of a nanoparticle according to any of claims 1 to 8 as selectin inhibitor.

15. Use of a nanoparticle according to claim 14 as inhibitor of L-selectin and/or P-selectin and/or E-selectin.
